# NEUE EUROPÄISCHE PATENTSCHRIFT

(11) **EP 0 769 516 B2**
(45) Veröffentlichungstag und Bekanntmachung des Hinweises auf die Entscheidung über den Einspruch: **22.04.2009**
(45) Hinweis auf die Patenterteilung: 11.02.2004
(21) Anmeldenummer: 96114436.7
(22) Anmeldetag: 10.09.1996
(51) Int. Cl.: C08J 5/12, A61J 1/05

(54) **Verbindungsanordnung und Verfahren zum Verbinden zweier Einzelteile eines medizinischen Systems**
Connection structure and process for connecting two single parts of a medical system
Arrangement de connexion et procédé pour la connexion de deux parties singulières d'un système médical

(30) Priorität: 16.09.1995 DE 19534413
(43) Veröffentlichungstag der Anmeldung: 23.04.1997
(73) Patentinhaber: Fresenius AG, 61352 Bad Homburg v.d.H. (DE)
(72) Erfinder: Heilmann, Klaus, 66606 St. Wendel (DE); Nicola, Thomas, 57350 Spicheren (FR)
(74) Vertreter: Luderschmidt, Schüler & Partner

(56) Entgegenhaltungen:
- EP-A- 0 495 729
- EP-A- 0 516 302
- EP-A- 0 619 998
- WO-A-82/04016
- WO-A-93/24568
- FR-A- 2 612 652
- GB-A- 2 177 974
- JP-A- 03 159 655
- US-A- 3 942 529
- US-A- 4 211 741
- US-A- 4 516 977
- US-A- 4 664 659
- US-A- 4 948 643
- US-A- 5 356 709
- 'Polymers: Chemistry & Physics', 1991, STANLEY THORNES Artikel J.M.G.COWIE, Seiten 252 - 255,298
- 'Medicals Device Technology, 1994', Artikel L. FISHER:POLYETHER BLOCK AMIDE TPES, Seiten 34 - 34,36
- 'Medical Device Technology', Artikel H.POL: 'The Future with SEBS-based compo', Seiten 18 - 20,22
- Datenblatt Eltex R P KS409

## Beschreibung

Die Erfindung befaßt sich mit dem Problem der dichten und festen Verbindung zweier Einzelteile, die zu einem medizinischen System gehören. Insbesondere betrifft die Erfindung Verbindungsanordnungen aus einem ersten und einem zweiten Einzelteil, bei welchen wenigstens ein Abschnitt einer Oberfläche aus einem ersten Kunststoffmaterial des ersten Einzelteils mit wenigstens einem Abschnitt einer Oberfläche aus einem zweiten Kunststoffmaterial des zweiten Einzelteils in einem Kontaktbereich, der zumindest Teile der sich berührenden Oberflächen des ersten und zweiten Einzelteils umfaßt, eine feste und dichte Verbindung miteinander bilden, welche durch Inkontaktbringen der miteinander zu verbindenden Oberflächen des ersten und zweiten Einzelteils, anschließende Wärmebehandlung der in Kontakt befindlichen Oberflächen der Einzelteile und nachfolgende Abkühlung erhältlich ist.

Ferner betrifft die Erfindung ein Verfahren zum Verbinden zweier Einzelteile, die zu einem medizinischen System gehören, mit dem Ziel der Herstellung einer dichten und festen Verbindung, wie es Gegenstand des Oberbegriffes des Anspruchs 15 ist.

Zum Stand der Technik werden die
US-A- 4,516,977 (= D1),
EP-A- 0 136 848 (= D2) und die
WO-A-82/04016 (= D3) genannt.

Verbindungsanordnungen oder Verbindungstechniken, mit denen sich die vorliegende Anmeldung auseinandersetzt, kennt man u.a. aus der D1.

Gegenstand der D1 ist ein medizinischer Beutel zur Aufbewahrung von Blut oder Infusionslösungen, bei dem ein Anschlußstück aus einem dimensionsstabilen Kunststoffmaterial in den Körper des Beutels aus einem unterschiedlichen Kunststoffmaterial eingesetzt ist, wobei zur Herstellung einer festen und fluiddichten Verbindungen zwischen Beutel und Anschlußstück eine Bindungsschicht aus unvernetztem Copolymer aus einem Olefin- und Vinylacetat oder Polyurethan zwischen Anschlußstück und den damit in Kontakt befindlichen Abschnitten des Beutelkörpers angeordnet ist.

Für den medizinischen Beutel gemäß der D1 werden Kunststoffmaterialien auf Polyolefinbasis vorgeschlagen, u.a. auf Basis von Polyethylen, -propylen und - butylen. Neben diesen Homopolymeren werden auch solche Kunststoffmaterialien in der D1 genannt, die substituierte Olefine darstellen, beispielsweise Methyl-, Ethyl-, Vinyl- oder Halogenatom-Substituenten tragen. Auch Copolymere oder Blends aus entsprechenden Materialien werden vorgeschlagen.

Als insbesondere bevorzugt für den Beutel werden Polyethylene mit mittlerer bis hoher Dichte, hohem Molekulargewicht und enger Molekulargewichtsverteilung angegeben. Ferner ist gemäß der D1 in diesem Fall darauf zu achten, daß die Polyolefine einen Schmelzpunkt unterhalb der Sterilisationstemperatur von 110 bis 120°C aufweisen.

Für die Anschlußstücke oder Inserts (gemäß D1) kommen relativ steife und transparente Kunststoffmaterialien in Frage, welche sich lediglich bei einem Druck von wenigstens 2 bar verformen lassen, zum Beispiel Hart-PVC, Polypropylen, Polyamid, Polycarbonat, Polyester, Polyacrylate und ähnliche und/oder auf den vorgenannten Verbindungen basierende Copolymere und styrolhaltige Copolymere, insbesondere werden aber Polycarbonate verwendet.

Materialien für die Bindungsschicht umfassen gemäß der D1 Copolymere aus einem Olefin mit Vinylacetat oder einem Polyurethan. Die Bindungsschicht ist zwischen 0,1 und 7 mm dick und wird zur Herstellung einer Verbindungstechnik zwischen Anschlußstutzen oder Insert und Beutel so angeordnet, daß eine Schlauchlänge des Bindungsschichtmaterials über das Äußere des Inserts oder Anschlußrohres gezogen oder gestreift wird, danach das Insert in eine dafür vorgesehene Öffnung des Beutels gesteckt und anschließend alles miteinander verschweißt wird.

Es ist auch möglich, die Bindungsschicht zur Fixierung zunächst mit dem Anschlußstück zu verschweißen, dann in die vorbereitete Anschlußöffnung des Beutels einzuführen und diesen zu versiegeln. In diesem Fall wird nach dem Verschweißen das verbundene System bei ungefähr 120°C und 2 bar autoklaviert; bei der Heißversiegelung selbst (Verschweißung) wird der Beutel mit dem Ethylen-Vinylacetat-Copolymer der Zwischenschicht verbunden, ebenso wie das Anschlußstück aus vorzugsweise Polycarbonat.

Die Verbindungstechnik gemäß D1 ist aufgrund mehrerer Umstände nachteilig. Einerseits ist das Zwischenlegen einer Verbindungsschicht zwischen die zu verbindenden Einzelteile eines Systems relativ umständlich. Andererseits ist der Beutel aufgrund der unterschiedlichen Materialien (die Bindeschicht ist nicht aus Polyolefin-Materialien) nicht recyclingfähig.

Eine weitere Verbindungstechnik ist Gegenstand der D2. Hieraus kennt man flexible Beutel aus Ethylen-Vinylacetat-Copolymeren (EVA), bei denen ein mehrschichtiger Anschlußschlauch aus Kunststoff so angeordnet wird, daß er mit dem Inneren des Beutels in Fließverbindung steht, wobei der Schlauch eine Außenschicht, die ein Ethylen-Vinylacetat-Copolymeres aufweist, welche mit der Oberfläche des Beutelinneren verbindbar ist, besitzt. Hierbei wird die Versiegelung bevorzugt mittels einer Hochfrequenzenergieversiegelung erreicht. Abgesehen davon, daß die mehrschichtigen Schläuche, Anschlußrohre oder -stutzen PVC-haltige Schichten aufweisen, wobei PVC ein nicht unbedenkliches Material bei der Entsorgung ist, und des weiteren fast immer Weichmacher enthält, die aus dem Material in die zu lagernde Lösung diffundieren können, ist ein Stoffgemisch aus PVC und Vinylacetat beim Recycling nur schwer trennbar.

Aus der D3 ist ein speziell zur Verbindung zweier unterschiedlicher Plastikmaterialien ausgebildeter Konnektor als Zwischenschicht bekannt, der sich besonders zur Verbindung von medizinischen Behältnissen, wie z.B. Blutbeuteln, mit flexiblen Schläuchen eignet, wenn die zu verbindenden Teile aus miteinander unverträglichen Materialien bestehen, so daß ein direktes Verschweißen der Einzelteile miteinander nicht möglich ist.

Beim Zwischenstück gemäß der D3 handelt es sich um einen coextrudierten oder spritzgegossenen Mehrschichtkonnektor mit Schichten aus unterschiedlichen Kunststoffmaterialien, wobei eine der Schichten bei einer niedrigeren Temperatur "schmilzt" als die andere.

Die D3 nennt dabei für das Material der inneren Konnektorschicht Polyvinylchlorid, während die äußere Schicht aus einem niedriger schmelzenden Polymer besteht, beispielsweise Polyethylvinylacetat (EVA). In dieser Ausführungsform eignet sich der Konnektor gemäß D3 z.B. zur Verbindung eines PVC-Schlauchs mit einem Polyolefin-Behältnis, dessen Anschlußstück aus einem Propyleneinheiten und Polyethylvinylacetateinheiten als erste und zweite Komponente aufweisenden Material besteht.

Zur Ausbildung der Verbindung wird der PVC-Schlauch nun mit der inneren Schicht des Konnektors, beispielsweise unter Verwendung eines Lösungsmittels wie Cyclohexanon, in Berührung gebracht. Die Außenschicht des Konnektors wird dann nach Verfestigung der inneren Schlauch-Konnektor-Anbindung, unter Reibungskontakt in das Anschlußelement des Beutels eingeführt. Die gesamte Schlauch-Konnektor-Anschluß-Beutel-Anordnung wird dann auf eine Temperatur gebracht, die ausreicht, das EVA der äußeren Schicht des Konnektors zu schmelzen und mit dem Anschlußstück zu verbinden. Diese Temperaturbehandlung kann u.a. in einem Autoklaven durchgeführt werden.

Zur Verbindung von Einzelteilen aus ähnlichen Materialien, und zur Erniedrigung der für deren Hitzeverbindung benötigten Temperatur schlägt die D3 die Verwendung zweier Konnektoren vor, einen ersten mit einer niedrigschmelzenden Kunststoff-Außenschicht (EVA) und einer höher-schmelzenden Kunststoff-Innenschicht (PVC) und einen zweiten mit der umgekehrten Schichtabfolge. Die beiden Konnektoren unterschiedlichen Durchmessers werden dann ineinandergesteckt, so daß die PVC-Schichten sich miteinander in Kontakt befinden. Die beiden PVC-Schichten werden anschließend miteinander lösungsmittelversiegelt (Cyclohexanon). Der resultierenden "Doppel-Konnektor" hat schließlich zwei niedrig-schmelzende Kunststoff-Außenschichten, die in bekannter Weise zur Verbindung von EVA-Beuteln und - Schläuchen einsetzbar sind.

Die aus der D3 für den Fachmann erkennbare Lehre ist gleich auf mehrfache Weise mit Nachteilen behaftet.

Einerseits ist jeweils mindestens ein zusätzliches Konnektorteil zur Verbindung eines medizinischen Systems (Beutel + Schlauch) notwendig. Andererseits werden letztlich immer wenigstens zwei Schritte zur Herstellung der Verbindung benötigt. So geht einer Hitzebehandlung regelmäßig eine Lösungsmittelverschweißung der PVC-Schichten des Konnektors und des Schlauches oder sogar zweier Konnektoren voraus, was insgesamt recht umständlich ist. Schließlich richtet sich die D3 ausschließlich auf klassische PVC- und EVA-Materialien, die unter heutigen Gesichtspunkten, vor allem vor dem Hintergrund der Weichmacherproblematik im Zusammenhang mit PVC, eher bedenklich erscheinen.

Angesichts des diskutierten Standes der Technik ist es mithin eine Aufgabe der Erfindung, eine Verbindungsanordnung oder Verbindungstechnik gemäß der eingangs erwähnten Art zur Verfügung zu stellen, die die Herstellung einer festen und fluiddichten Verbindung zwischen zwei oder auch mehreren, zu einem System gehörenden Einzelteilen ermöglicht. Die Verbindungsanordnung soll aus PVC-freien und vinylfreien Materialien sein, frei von Weichmachern, Haftvermittlern oder sonstigen Zusatzstoffen. Vor allem aber soll die Verbindungstechnik auch ohne zusätzliche Konnektoren oder Verbindungsteile oder dergleichen für die Verbindung zweier zu einem medizinischen System gehörender Einzelteile, wie etwa Schlauch und Konnektionsstelle, auskommen können. Aufgabe der Erfindung ist auch die Angabe eines Verfahrens zum Verbinden zweier Einzelteile eines medizinischen Systems, ohne zusätzliche Hilfsmittel nur durch Hitzebehandlung. Gleichzeitig müssen jedoch alle Einzelteile formstabil sein.

Gelöst werden diese und weitere nicht im einzelnen angegebene Aufgaben durch eine Verbindungsanordnung der eingangs erwähnten Gattung mit den Merkmalen des kennzeichnenden Teils des Anspruchs 1. Vorteilhafte Ausgestaltungen der Erfindung sind Gegenstand der auf Anspruch 1 rückbezogenen Ansprüche.

In verfahrensmäßiger Hinsicht stellt der Gegenstand des Anspruchs 13 eine Lösung des der Erfindung zugrunde liegenden Problems dar. Vorteilhafte erfindungsgemäße Verfahrensabwandlungen werden in den abhängigen Verfahrensansprüchen unter Schutz gestellt.

Dadurch, daß das erste Kunststoffmaterial wenigstens ein Polymer aufweist, welches bei der Temperatur der Wärmebehandlung formbeständig ist, während das zweite Kunststoffmaterial wenigstens ein Polymer aufweist, welches bei der Temperatur der Wärmebehandlung nicht mehr formbeständig und unter Einwirkung einer Preßkraft, also unter Druck, fließfähig ist, d. h. zum Fließen neigt, wobei beide Kunststoffmaterialien frei von PVC oder EVA sind und die Verbindung durch Inkontaktbringen der Oberflächen des ersten und zweiten Einzelteils unter gleichzeitiger Einwirkung einer die Oberflächen gegeneinander pressenden Kraft und Wärmebehandlung der gegeneinander gepreßten Einzelteile erhältlich ist, wobei die Wärmebehandlung ein Hitzesterilisationsvorgang ist und wobei die Temperatur der Wärmebehandlung ≥121°C ist und wobei der Non-PVC-Mehrschichtschlauch für medizinische Zwecke wenigstens zwei Schichten aufweist, von denen eine Basisschicht A) aus einem dritten Kunststoffmaterial mit wenigstens einer Konnektionsschicht B) aus dem zweiten Kunststoffmaterial verbunden ist, wobei das dritte Kunststoffmaterial wenigstens ein Polymer aufweist, dessen Formbeständigkeit größer als die Temperatur der Wärmebehandlung ist, während das zweite Kunststoffmaterial wenigstens ein Polymer aufweist, welches unter Konnektionsdruck Fließfähigkeit bei einer Temperatur kleiner oder gleich der Temperatur der Wärmebehandlung aufweist, und wobei in einer besonderen Ausführungsform der Konnektor, das Einbauteil oder der Anschlußstutzen (erstes Einzelteil) wenigstens im.

Kontaktbereich aus Polypropylen (PP), Polycarbonat (PC), einem die Struktureinheiten, die den vorgenannten Homopolymeren zugrundeliegen, aufweisenden Copolymeren und/oder aus Blends, die auf den vorgenannten Polymeren basieren, mit bis zu 40% Gew.-Anteil bezogen auf das Gesamtgewicht des Kunststoffmaterials des ersten Einzelteils, des zweiten Kunststoffmaterials besteht, wobei das zweite Kunststoffmaterial PE-Copolymer, SEBS/SEPS mit Diblockanteil und/oder Polyetherblockamid ist, gelingt es vorteilhaft eine Verbindungsanordnung bereitzustellen, die
1. vollständig auf Materialien wie PVC oder EVA verzichtet;
2. auf einfache Weise die dichte, sterile, feste und dauerhafte Verbindung zwischen einem Konnektor, Einbauteil oder Anschlußstutzen (erstes Einzelteil) eines medizinischen Systems und einem Non-PVC-Mehrschichtschlauch (zweites Einzelteil) für medizinische Zwecke während einer einfach durchzuführenden Wärmebehandlung gestattet; und zwar
3. ohne daß dabei umständliche zusätzliche Konnektoren in Form von Verbindungs- oder Adapterschläuchen benötigt würden (zusätzliche Konnektoren sind jedoch möglich) und
4. ohne daß dabei weitere Versiegelungstechniken, wie etwa die Verwendung von Lösungsmitteln, zusätzlich anzuwenden wären.

Vorteilhafterweise ergibt sich die stabile Verbindung bei der in medizinischen Systemen routinemäßig angewendeten Heißsterilisationsverfahren, so daß kein Zusatzschritt notwendig wird.

Die erfindungsgemäße Verbindungsanordnung ist Bestandteil eines medizinischen Systems. Hierunter werden im Rahmen der Erfindung im weitesten Sinne alle auf dem medizinisch-technischen Sektor eingesetzten Behältnisse, Gefäße, Schläuche, Beutel und zugehörigen Verbindungselemente verstanden, die bei sachgerechtem und bestimmungsbedingtem Einsatz mit Körperflüssigeiten wie Blut oder zur therapeutischen Behandlung des menschlichen Körpers gedachten Flüssigkeiten, wie etwa Infusionslösungen, in Kontakt kommen können.

Dabei umfaßt die Verwendung des Begriffes "System" gemäß der Erfindung ferner eine Anordnung von wenigstens zwei Einzelteilen oder Elementen, die zum Einsatz des Systems fest und sicher miteinander verbunden sein sollen, so daß keine Gefahr für das einwandfreie Funktionieren des Systems besteht.

Das erste Einzelteil besteht in der vorliegenden Erfindung aus einem Konnektor, Einbauteil oder Anschlußstutzen eines medizinischen Systems und das zweite Einzelteil besteht aus einem Non-PVC-Mehrschichtschlauch für medizinische Zwecke. Nachfolgend werden nur noch die Begriffe "erstes Einzelteil" und "zweites Einzelteil" gebraucht, wobei diese die zuvor genannten Bedeutungen haben.

Zu beispielhaften medizinischen Systemen gehören u.a. medizinische Beutelsysteme für Lösungen, die mit Konnektoren, Einbauteilen oder Anschlußstutzen ausgerüstet sind und mit Schläuchen, Schlauchsystemen oder Schlauchsets zu verbinden sind, um den Transport der medizinischen Lösungen zur therapeutischen Behandlung zu gewährleisten; ferner zählen zu den medizinischen Systemen Schlauchsets selbst, die die Verbindung von Schläuchen zu mehreren untereinander erforderlich machen, beispielsweise mit für diesen Zweck geeigneten Konnektoren oder allen anderen, dem Fachmann auf dem Medizintechnik-Sektor geläufigen und eingesetzten Elemente.

Damit nun im Rahmen der Erfindung zwischen zwei zu einem System gehörenden Einzelteilen eine feste und dichte Verbindung ausgebildet ist, weisen die gemäß der Erfindung zu verbindenden Einzelteile zumindest Oberflächen auf, die wenigstens teilweise aus Kunststoff bestehen. Dabei genügt es, daß wenigstens bestimmte Abschnitte der jeweiligen Oberflächen der Einzelteile aus Kunststoffmaterialien bestehen, wobei die bestimmten Abschnitte sich zur Ausbildung der Verbindung miteinander in Kontakt befinden. Die miteinander in Kontakt befindlichen Oberflächen der Einzelteile definieren einen Kontaktbereich, wobei sich versteht, daß der Kontaktbereich nicht die sich berührenden Oberflächen des ersten und zweiten Einzelteils im allgemeinen umfaßt, sondern im speziellen die sich berührenden Abschnitte aus Kunststoff, wenn die Einzelteile des Systems in derjenigen Position angeordnet sind, in welcher die Verbindung ausgebildet werden soll. Es bleibt also festzuhalten, daß der Kontaktbereich auf die sich berührenden Oberflächenabschnitte aus Kunststoff beschränkt ist, während die sich berührenden Oberflächenabschnitte des ersten und zweiten Einzelteils selbst über den Umfang des hierin definierten Kontaktbereiches hinausgehen können.

Die feste und dichte Verbindung der Einzelteile ist erfindungsgemäß durch Inkontaktbringen der Oberflächen unter gleichzeitiger Einwirkung einer die Oberflächen gegeneinander pressenden Kraft, Wärmebehandlung der gegeneinander gepreßten Einzelteile und anschließende Abkühlung erhältlich.

Hierunter ist zu verstehen, daß die Einzelteile beispielsweise durch In- oder Aufeinanderstecken einen Preßsitz erhalten, der die sich berührenden Oberflächen der Einzelteile mit einer Kraft gegeneinander preßt, die zum einen eine gewisse Formstabilität der Anordnung aus den zu verbindenden Einzelteilen vor Ausbildung der festen und dauerhaften Verbindung gewährleistet und zum anderen die den Kontaktbereich ausbildenden Oberflächenabschnitte der Einzelteile in innigem Materialkontakt der sich berührenden Oberflächen miteinander fixiert.

Dabei wird in der Erfindung unter einer gleichzeitigen Einwirkung einer die Oberflächen gegeneinander pressenden Kraft vor allem die Tatsache verstanden, daß zumindest eines der zu verbindenden Einzelteile durch eine Last oder Kraft deformiert wird (beim Ineinanderstecken), aber nach Entfernen der Last oder Kraft bestrebt ist, in seine Ausgangsform zurückzukehren. Dieses "elastische" Verhalten ist erfindungsgemäß von besonderem Vorteil, wobei die vorherige Deformation auf jede dem Fachmann geeignet erscheinende Weise herbeigeführt werden kann. Möglich sind u.a. Kompression, Stauchung, Biegung, Verschiebung, Verdrillung und dergleichen.

Der Erfindung liegt nun der Gedanke zugrunde, durch geeignete Wahl und Abstimmung der die Kunststoffmaterialien im Kontaktbereich aufbauenden Polymeren unter gleichzeitigem gänzlichem Verzicht auf PVC oder EVA eine Verbindungsanordnung zu schaffen, die in einem einzigen Wärmebehandlungsvorgang fest und sicher ausgebildet werden kann, ohne daß zusätzliche Haft-, Dichtungs- oder Versiegelungsmassen oder Hilfsmittel einzusetzen wären oder andere Versiegelungsverfahren, wie etwa hochfrequente Energie und dergleichen. Insbesondere ist es Grundgedanke der Erfindung, eine feste Verbindung eines Konnektors oder Einbauteils mit einem Schlauch in der Art zu schaffen, daß die Kontaktbereiche des Schlauches und des Konnektors oder Einbauteils aus Materialien bestehen oder zu gewissen Anteilen Materialien enthalten, die sich bei einer nachfolgenden Wärmebehandlung, insbesondere einer Hitzesterilisation und darauffolgender Abkühlung fest miteinander verbinden.

Für die Erfindung werden unter "Kunststoffmaterial" solche Materialien verstanden, deren wesentliche Bestandteile aus makromolekularen organischen Verbindungen bestehen, wobei die Kunststoffmaterialien ein oder mehrere Polymere aufweisen können oder auch einfach als Polymere bezeichnet werden können, wobei zu Polymeren insbesondere Homopolymere als auch Copolymere (auch statistische, Block- und/oder Pfropfpolymere) sowie Mischungen (= Blends) aus den genannten Stoffen gehören.

Ein wichtiges Kriterium für die erfindungsgemäße Auswahl und Zuordnung eines Polymers zu einem Kunststoffmaterial sowohl beim ersten als auch beim zweiten Einzelteil (und zwar jeweils im Kontaktbereich), stellt die Formbeständigkeit unter Sterilisationsbedingungen (Fließfähigkeit) des Polymers dar. Die Formbeständigkeit hängt im wesentlichen von der Erweichungstemperatur (von der Vicat-Temperatur), von der Materialhärte und vom E-Modul der eingesetzten Stoffe ab.

Die Erweichungstemperatur wird für die Polymere und Kunststoffmaterialien der Erfindung nach Vicat bestimmt, d.h. sie ist als diejenige Temperatur definiert, bei der in eine zunehmend erwärmte Kunststoffprobe ein definiert belasteter Stahlstift von 1 mm² Querschnitt 1 mm tief eingedrungen ist (alt: DIN 53 460 364, neu DIN-ISO 306).

Dadurch, daß nun das erste Kunststoffmaterial des 1. Verbindungsteils gemäß der Erfindung ein Polymer aufweist, dessen Formbeständigkeit bei Temperaturen größer als die Temperatur einer Wärmebehandlung gegeben ist, wird die notwendige Formstabilität beim Vorgang der Verbindungsbildung gewährleistet, während dadurch, daß das zweite Kunststoffmaterial ein Polymer aufweist, das unter Konnektionsdruck fließfähig und bei Temperaturen kleiner oder gleich der Temperatur der Wärmebehandlung nicht mehr formbeständig ist, die eigentliche Verbindungsbildung ermöglicht wird. Im Rahmen der Erfindung haben sich für das 1. Einzelteil dabei Polypropylen, Polycarbonat, sowie deren Copolymere oder Blends die auf den genannten Homo- oder Copolymeren basieren mit bis zu 40% des Polymers des 2. Einzelteils, sowie PE-Copolmer, SEBS/SEPS mit Diblockanteil oder Polyetherblockamid mit einer Härte von Shore D ≤ 32 für das 2. Einzelteil als besonders günstig erwiesen, um die Verbindungsanordnung auszubilden. Weiterhin zweckmäßig ist es, wenn das erste Einzelteil ein Blend mit bis zu 40 Gew.-% Anteil des Polymers des zweiten Einzelteils ist und umgekehrt.

Grundsätzlich reicht es zwar im Rahmen der Erfindung aus, wenn die Kunststoffmaterialien die entsprechenden genannten Polymeren aufweisen, die Erfindung kann jedoch mit besonders gutem Erfolg verwirklicht werden, wenn die Kunststoffmaterialien nicht nur Polymere aufweisen, deren Formbeständigkeit den vorgenannten Kriterien entsprechen, sondern wenn die Formbeständigkeiten der Kunststoffmaterialien selbst bei Temperaturen größer bzw. kleiner oder gleich der Wärmebehandlung gegeben oder nicht mehr gegeben sind. In diesem bevorzugten Fall weist das Kunststoffmaterial die gewünschte Eigenschaft nicht nur partiell in Form eines Bestandteiles seines Materials auf, vielmehr verfügt das gesamte Material über das angestrebte Eigenschaftsprofil.

In einer Ausführungsform enthält das 1. Einzelteil bis zu 40 Gew.-% des Polymers des 2. Einzelteils. Hierdurch wird eine bessere Haftung erzielt, sofern die Materialviskositäten so abgestimmt sind, daß sich Teile des Polymers des 2. Einzelteil an der Oberfläche des 1. Einzelteils befinden.

Daher kann man die Erfindung im Rahmen der Ansprüche prinzipiell an eine Vielzahl von Wärmebehandlungen und die damit verbundenen Temperaturen "anpassen".

Die Temperatur, die nun für die Erweichung der erfindungsgemäßen Polymere oder Kunststoffmaterialien von besonderem Interesse ist, also die Temperatur der Wärmebehandlung, ist diejenige Temperatur, unter der üblicherweise die Dampfsterilisation vorgenommen wird. Hierbei wird im Rahmen der Erfindung unter Dampfsterilisation allgemein ein Verfahren für die Abtötung bzw. Inaktivierung (Viren) aller Mikroorganismen einschließlich der hochresistenten Dauerformen verstanden, wobei die erfindungsgemäßen Materialien insbesondere einer Dampfsterilisation im Autoklaven mit Wasserdampf von mindestens 121°C, entsprechend etwa einer Atmosphäre Überdruck, der sogenannten Autoklavierung oder Autoklavierungsbehandlung unterzogen werden können, ohne Schaden zu nehmen.

Wenn man also die Verbindungsausbildung unter den Standardbedingungen der Dampfsterilisation zuläßt, kann man die Sterilisation und Verbindungsbildung bei der Anordnung gemäß der Erfindung in einem einzigen Vorgang bewirken. Dabei ist bevorzugt, daß das Polymer oder Kunststoffmaterial des zweiten Einzelteils nur dort erweicht, also in einen fließfähigen Zustand übergeht, wo es einer Preßkraft ausgesetzt ist. An Stellen ohne zusätzliche Krafteinwirkung wird der fließfähige Zustand nicht ausreichend erreicht. In einem solchen Fall wäre nämlich die Aufgabe der Verbindungsanordnung nicht mehr erfüllbar, eine genügend feste, dichte und widerstandsfähige Verbindung mit einem anderen Kunststoffmaterial eines Verbindungspartners einzugehen, ohne die Formstabilität des Schlauches oder des Körpers der Verbindungspartner an nicht zu verbindenden Bereichen unkontrollierbar werden zu lassen.

Die Verbindungsanordnung gemäß der Erfindung kann wie bereits erwähnt, Einzelteile umfassen und verbinden, bei denen nur bestimmte Abschnitte aus Kunststoffmaterial bestehen. In vorteilhafter Ausbildung zeichnet sich die Verbindungsanordnung der Erfindung allerdings dadurch aus, daß die verbundenen Einzelteile vollständig aus Kunststoff bestehen. Dies läßt insbesondere eine Fertigung der Einzelteile nach dem vorteilhaften Verfahren der Kunststoffverarbeitungstechnik zu und ist des weiteren insgesamt vorteilhaft für den Einsatz auf medizinischem Sektor. In zweckmäßiger Ausgestaltung der erfindungsgemäßen Verbindungsanordnung besteht das erste Einzelteil wenigstens im Kontaktbereich aus einem Kunststoffmaterial oder Blend von Kunststoffmaterialien, aufweisend
a) 60 - 100 Gew.- % Polypropylen oder Polycarbonat
   und
b) 40 - 0 Gew.- % Polyethylen-Copolymer oder SEBS/SEPS mit Diblockanteil oder Polyetherblockamid.

Bevorzugt sind 30 - 10 Gew.-% der Komponente b).
Ganz besonders bevorzugt besteht das erste Einzelteil wenigstens im Kontaktbereich aus Polypropylen.

Hinsichtlich des zweiten Einzelteils gilt, daß es sich dabei um einen Non-PVC-Mehrschichtschlauch für medizinische Zwecke handelt, wobei dieser wenigstens zwei Schichten aufweist, von denen eine Basisschicht A) aus einem dritten Kunststoffmaterial mit wenigstens einer Konnektionsschicht B) aus dem zweiten Kunststoffmaterial verbunden ist, wobei das dritte Kunststoffmaterial wenigstens ein Polymer aufweist, dessen Formbeständigkeit größer als die Temperatur der Wärmebehandlung ist, während das zweite Kunststoffmaterial wenigstens ein Polymer aufweist, welches unter Konnektionsdruck Fließfähigkeit bei einer Temperatur kleiner oder gleich der Temperatur der Wärmebehandlung aufweist. Hierdurch gelingt es besonders zweckmäßig, einen flexiblen Schlauch, der während der Hitzesterilisation thermisch stabil und nach erfolgter Hitzesterilisation transparent ist, der ausreichende Knickstabilität aufweist und der mit Schlauchklemmen oder dergleichen abdichtbar ist mit einem ersten Einzelteil in Form eines Einbauteils, eines medizinischen Beutels oder eines Konnektors fest und dicht zu verbinden und zwar während einer Hitzesterilisationsbehandlung.

Während häufig schon bei Vorliegen von nur untergeordneten Massenanteilen an Polymeren mit dem gewünschten Erweichungsverhalten die erfindungsgemäße Verbindungsbildung bei gleichzeitig gewährleisteter Stabilität und Flexibilität des Schlauches im Rahmen der Erfindung erreichbar sind, wird dadurch, daß der überwiegende Teil des Kunststoffmaterials oder Polymers jeder einzelnen Schicht des Schlauches Träger der gewünschten Eigenschaft ist, das Gesamtverhalten des Schlauchmaterials verbessert und die Gefahr ausgeschlossen, daß es nicht zu einer ausreichenden Verbindungsausbildung während einer Dampfsterilisation kommt. Daher kennzeichnet sich die Erfindung dadurch, daß die Basisschicht A) bei Temperaturen > 121 °C deformierbar erweicht und jede Konnektionsschicht B) bei Temperaturen ≤ 121°C deformierbar unter Konnektionsdruck erweicht.

Der erfindungsgemäße Schlauch kann z.B. auf einen Konnektor oder Stutzen eines Bauteils (als erstes Einzelteil) aus geeignetem Material "aufgesteckt" werden, so daß die Innenschicht des Schlauchs mit der Außenoberfläche des Konnektors in Berührung ist oder zum Einführen in ein Hohlteil gedacht sein, dessen Innenoberfläche aus geeignetem Material zur Ausbildung einer Verbindung besteht, während bei Anordnung zweier Verbindungsschichten (außen und innen) beim Einzelteil "Non-PVC-Mehrschichtschlauch" die beiden genannten Verbindungsmöglichkeiten wahlweise oder gleichzeitig realisierbar sind.

Hinsichtlich der Ausbildung der Verbindung mit anderen Bauteilen, wie Beuteln, Konnektoren, Anschlußstutzen oder ähnlichem ist grundsätzlich anzumerken, daß es zur Ausbildung einer dichten und stabilen Verbindung nicht nur des Einflusses der Temperatur der Hitzesterilisation bedarf, sondern daß, wie bereits ausgeführt, auch eine andere Einflußgröße eine wesentliche Rolle spielt. Und zwar ist dies der Preßdruck, mit dem die beiden zu verbindenden Flächen sich während der Temperatureinwirkung der Hitzesterilisation berühren. Für die Verwirklichung der Erfindung ist es daher wesentlich, daß das Einzelteil "Non-PVC-Mehrschichtschlauch" einen Preßsitz mit einem Einzelteil "Konnektor oder Anschlußstutzen eines Beutels" ausbildet, so daß es während des Dampfsterilisierens durch Kraft- und Formschluß unterstützt (Preßkraft) zu einem Angelieren der zur Verbindungsausbildung befähigten Schichten kommt, wobei die Haftung einerseits durch ein Verschmelzen von erweichten Materialschichten beim innigen Kontakt erreicht wird und andererseits durch eine Oberflächenhaftung bei Wahl geeigneter Oberflächen, beispielsweise solcher, mit besonders rauhem Profil, sichergestellt wird.

Beim erfindungsgemäß bevorzugten Einzelteil "Non-PVC-Mehrschichtschlauch" ist die Verbindungsschicht B, welche zur Verbindung zu dem Konnektor zeigt, vorteilhaft zum überwiegenden Teil ein Styrol Ethylen Butylen oder (Propylen)-Kautschuk mit Diblockanteil oder PE-Copolymer, während die Basisschicht A bevorzugt zum überwiegenden Teil ein Polypropylen oder SIS ist. Diese Materialkombinationen für die Basisschicht A) und die Konnektionsschicht oder -schichten B) kann bereits eine Vielzahl an geforderten Eigenschaften bieten. Für die Erfindung besonders vorteilhaft ist die Basisschicht A) ein Blend, aufweisend bezogen auf die Gesamtmenge des dritten Kunsstoffmaterials in Gew.-%

| | | |
|---|---|---|
| a) | 40%-100% PE-Copolymer | (Exxact 4022, Exon) |
| | 0%-60% SEBS/SEB | (Kraton G 1726, Shell), |
| b) | 40%-100% PE-Copolymer | (Engage XU 58.000.52, Dow Chemical) |
| | 0%-60% SEBS/SEB | (Kraton G 1726, Shell), |
| c) | 100% SEPS/SEP | (Septon 2277, Kuraray), |
| d) | 40%-100% SEBS/SEB | (Kraton G 1726, Shell) |
| | 0-60% SEBS | (Kraton G 1652, Shell) |
| | oder | |
| e) | 100% Polyetherblockamid | (Pebax 35335SA, ATO). |

Dabei handelt es sich bei SEBS/SEB (Kraton G 1726) um ein niedermolekulares SEBS mit mindestens 20% Diblock-Anteil.

Ein Schlauch mit einer der oben beschriebenen Konnektionsschichten ist bevorzugt mit einem Konnektor aus Polypropylen-Random-Copolymer oder Polypropylen - Homopolymer verbindbar, während Polyetherblockamide mit Polycarbonat verbindbar sind.

Grundsätzlich ist es im Rahmen der Erfindung bevorzugt und in der Regel auch gewährleistet, daß die verschiedenen Schichten B) und A) trotz eines Gehalts an zur Stabilisierung üblichen Anteilen im wesentlichen frei von Weichmachern, Antiblockmitteln, Antistatika oder anderen Füllstoffen sind. So wird insbesondere dem von PVC bekannten Weichmacherproblem begegnet.

Die Konnektionsschicht B) kann auch beidseitig der Basisschicht A) sein. Ferner kann die Konnektionsschicht B) 30 Gew.-% des Materials der Schicht A) aufnehmen und umgekehrt. Durch diese "Materialvermittlung" bzw. die Substitution von Material wird die Kompatibilität der miteinander beispielsweise zu einem Schlauch geformten Schichten deutlich gesteigert, ohne die sonstigen Eigenschaften in Frage zu stellen.

Ein weiteres besonders bevorzugtes Merkmal der Erfindung ist darin zu sehen, daß in erfindungsgemäßer Weiterbildung der bekannten Verbindungsanordnungen, die Kunststoffmaterialien für alle Einzelteile der Anordnung so gewählt sind, daß sie im wesentlichen aus Poylolefin-Homopolymeren oder Polyolefin-Copolymeren bestehen. Es war insbesondere erstaunlich, daß es mit der Erfindung erstmals gelingt, eine Verbindungstechnik, also eine Verbindung eines medizinischen Beutels mit beispielsweise einem Non-PVC-Mehrschichtschlauch zu schaffen, welche ausschließlich aus leicht recycelbaren Materialien besteht, welche die problemlose Verbindungsbildung miteinander oder mit Konnektoren aus umweltfreundlichen Materialien bei der Dampfsterilisation gestattet und gleichzeitig alle weiteren Forderungen an ein medizinisch einsetzbares System erfüllt.

Gegenstand der Erfindung ist auch ein Verfahren zum Verbinden zweier zu einem medizinischen System gehörender Einzelteile ohne zusätzliche Hilfsmittel nur durch Hitzebehandlung, bei welchen unter Ausbildung eines Kontaktbereiches, der durch sich berührende Flächen des ersten und zweiten Einzelteils definiert ist, wenigstens ein Abschnitt einer Oberfläche des ersten Einzelteils aus einem ersten Kunststoffmaterial mit wenigstens einem Abschnitt einer Oberfläche des zweiten Einzelteils aus einem zweiten Kunststoffmaterial zu einer Verbindungsanordnung zusammengefügt wird, anschließend die zusammengefügte Verbindungsanordnung zur Ausbildung einer festen und dichten Verbindung zwischen erstem und zweitem Einzelteil einer Wärmebehandlung unterzogen und nachfolgend abgekühlt wird, wobei die Erfindung dadurch gekennzeichnet ist, daß man ein erstes Kunststoffmaterial verwendet, das wenigstens ein Polymer aufweist, dessen Formbeständigkeit bei der Temperatur der Wärmebehandlung gegeben ist, während man ein zweites Kunststoffmaterial verwendet, das wenigstens ein Polymer aufweist, welches bei der Temperatur der Wärmebehandlung nicht mehr formbeständig ist und zum Fließen neigt, wobei beide Kunststoffmaterialien frei von PVC sind und wobei erstes und zweites Einzelteil unter Ausbildung eines Preßsitzes zusammengefügt werden, wobei das Polymer des zweiten Kunststoffmaterials bei der Temperatur der Wärmebehandlung nicht mehr formbeständig ist und unter Einwirkung einer die Oberflächen gegeneinander pressenden Kraft (Konnektionsdruck oder - kraft) zum Fließen neigt, wobei die Wärmebehandlung ein Hitzesterilisationsvorgang ist und wobei die Temperatur der Wärmebehandlung ≥121°C ist und wobei es sich beim zweiten Einzelteil um einen Non-PVC-Mehrschichtschlauch handelt, wobei der Non-PVC-Mehrschichtschlauch für medizinische Zwecke wenigstens zwei Schichten aufweist, von denen eine Basisschicht A) aus einem dritten Kunststoffmaterial mit wenigstens einer Konnektionsschicht B) aus dem zweiten Kunststoffmaterial verbunden ist, wobei das dritte Kunststoffmaterial wenigstens ein Polymer aufweist, dessen Formbeständigkeit größer als die Temperatur der Wärmebehandlung ist, während das zweite Kunststoffmaterial wenigstens ein Polymer aufweist, welches unter Konnektionsdruck Fließfähigkeit bei einer Temperatur kleiner oder gleich der Temperatur der Wärmebehandlung aufweist. In besonders bevorzugter Verfahrensabwandlung besteht das erste Einzelteil wenigstens im Kontaktbereich aus Polypropylen (PP), Polycarbonat (PC), einem die Struktureinheiten, die den vorgenannten Homopolymeren zugrunde liegen, aufweisenden Copolymeren und/oder aus Blends, die auf den vorgenannten Polymeren basieren, vorteilig mit bis zu 40 Gew.-% des Polymers des 2. Einzelteils. Das zweite Einzelteil setzt sich in diesem Fall bevorzugt aus PE-Copolymer, SEBS/SEPS mit Diblockanteil oder Polyetherblockamid zusammen.

In weiterhin bevorzugten Verfahrensvarianten der Erfindung werden Einzelteile die zu einem Beutelsystem für medizinische Lösungen gehören oder solche Einzelteile, die zu einem medizinischen Schlauchsystem gehören, miteinander verbunden. Außerdem ist es vorteilhaft, daß man als erstes Einzelteil einen Konnektor oder ein Einzelteil aus Kunststoff und als zweites Einzelteil einen coextrudierten Non-PVC-Mehrschichtschlauch miteinander verbindet, wobei in allen Fällen als Wärmebehandlung einen Sterilisationsvorgang unter den an sich bekannten Bedingungen vorgenommen wird. Die Wärmebehandlung wird bevorzugt bei einer Temperatur von 121°C durchgeführt. Je nach angestrebter Temperatur kann die Eigenschaft der zu verbindenden Materialien der beiden Einzelteile gezielt verändert werden, etwa durch Auswahl der verwendeten Kunststoffmaterialien.

So ist es im Rahmen der Ansprüche von überaus großem Vorteil im Verfahren der Erfindung ein erstes Kunststoffmaterial zu verwenden, dessen Formbeständigkeit bei Temperaturen größer als 121°C gegeben ist, während man ein zweites Kunststoffmaterial verwendet, das bei Temperaturen kleiner oder gleich 121°C unter Preßdruck oder Konnektionsdruck fließfähig ist.

In zweckmäßiger Verfahrensmodifikation kennzeichnet sich das Verfahren der Erfindung dadurch, daß man einen coextrudierten Non-PVC-Mehrschichtschlauch als eines der Einzelteile verwendet, dessen Innenund/oder Außenschicht aus mindestens 30 Gew.-% SEBS (SEPS) mit Diblockanteil besteht, während der Konnektor oder das Einbauteil selbst aus Polypropylen besteht.

Weiterhin kann es vorteilhaft sein, daß man einen coextrudierten Non-PVC-Mehrschichtschlauch dessen Konnektionsschicht aus Polyethylen-Copolymer, SEPS oder SEBS mit Diblockanteil oder Gemischen daraus besteht und einen Konnektor oder Einbauteile verwendet, das aus Polypropylen besteht oder mit Polyethylen-Copolymer oder Styrol-Ethylen-Butylen (Propylen)-Kautschuk gemischtes Polypropylen.

Schließlich ist es auch bevorzugt, daß man einen coextrudierten Non-PVC-Mehrschichtschlauch, dessen Innen- und/oder Außenschicht aus Polyetherblockamid besteht und einem Konnektor oder ein Einbauteil miteinander verbindet, welche aus Polycarbonat bestehen.

Im Folgenden wird die Erfindung anhand von Ausführungsbeispielen unter Verweis auf die beigefügten Figuren weitergehend erläutert.

In den Figuren zeigen
- Fig. 1: einen Querschnitt durch eine Ausführungsform eines zweiten Einzelteils einer erfindungsgemäßen Verbindungsanordnung, wobei das zweite Einzelteil einen Non-PVC-Mehrschichtschlauch darstellt;
- Fig. 2: einen Längsschnitt durch eine Ausführungsform einer erfindungsgemäßen Verbindungsanordnung aus erstem und zweitem Einzelteil, wobei das erste Einzelteil einen Stutzen, Konnektor bzw. Einbauteil darstellt, während das zweite Einzelteil einen Schlauch repräsentiert; und
- Fig. 3: einen Längsschnitt durch eine weitere Ausführungsform der erfindungsgemäßen Verbindungsanordnung.

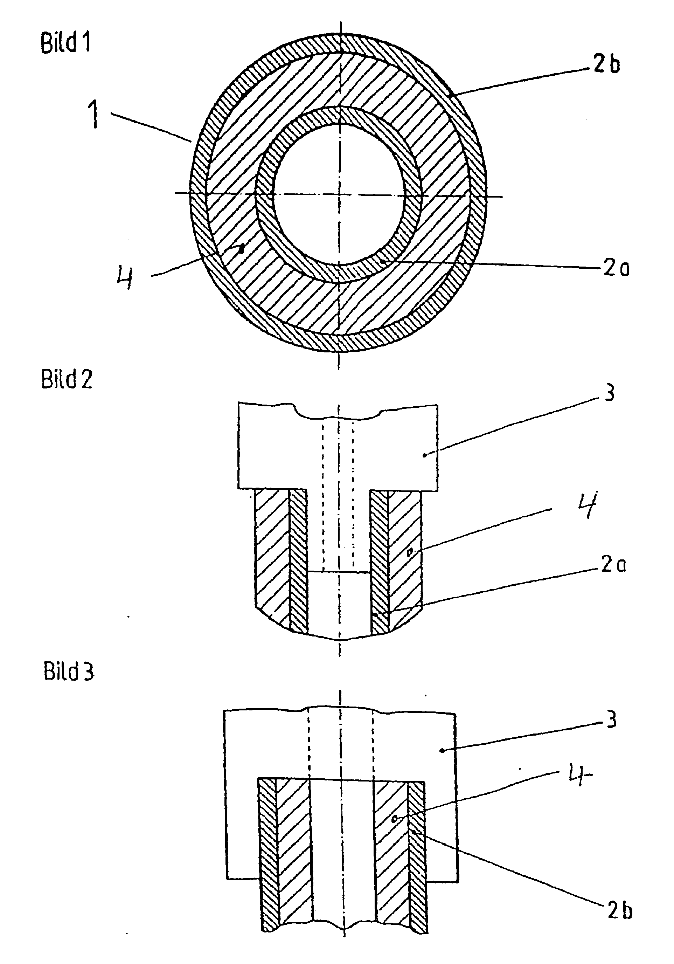

Bei dem in Fig. 1 dargestellten Schlauch 1 handelt es sich um einen coextrudierten Non-PVC-Mehrschichtschlauch mit drei Schichten. Die Schlauchschichten sind aus Polyolefinen, synthetischen Kautschuken oder Kombinationen daraus aufgebaut. Erfindungsgemäß stellt die Innenschicht 2a und/oder Außenschicht 2b des Schlauches, abhängig davon ob eine Schlauchinnen - und/oder Schlauchaußenverbindung gewünscht ist, die Verbindungsschicht mit einem speziellen Material oder einer besonderen Materialkombination dar, die die Fähigkeit besitzt bei einer entsprechenden nachfolgenden Wärmebehandlung und darauf folgenden Abkühlung eine feste Verbindung einzugehen. Vorzugsweise ist die Verbindungsschicht 2a und/oder 2b eine sehr dünne Schicht und die Formbeständigkeit der Schicht oder von Teilen der Schicht liegt bei einwirkendem Preßdruck unterhalb der Sterilisationstemperatur. Besonders geeignete Materialien für die Verbindungsschicht 2a und/oder 2b sind u.a. Styrol-Ethylen-Butylen (od. Propylen)-Kautschuke, Polyether Blockamid, PE-Copolymer, oder Blends daraus. In der gezeigten dreischichtigen Variante des Schlauches 1 entspricht die Schicht 4 der in der Beschreibung genannten Basisschicht (A).

Für die als Einzelteil der erfindungsgemäßen Verbindungstechnik fungierenden Schläuche 1 kommen bevorzugt solche in Frage, die hinsichtlich ihrer Einzelschichten folgende Formbeständigkeiten aufweisen:

| | E-Modul | Härte | Formbeständigkeit bei Hitzeeinwirkung |
|---|---|---|---|
| Hauptschicht Basis (A) =Schicht 4 | ≤80N/mm² | Shore D≤32 | >121°C |
| Konnektionsschicht (B) = Schicht 2a, 2b | ≤80N/mm² | Shore A≤65 | ≤121°C |
| optionell Deckschicht (C) | <1000 N/mm² | <R90 | >121°C |

Wie in den Figuren 2 und 3 dargestellt wird, stellt der Konnektor 3 (Fig.2) bzw. das Einbauteil 3 (Fig.3) mit dem Schlauch 1 durch die feste Verbindung ein dichtes System dar. Dabei ist aus Vereinfachungsgründen bei der in Fig. 2 dargestellten Ausführungsform die Konnektions-oder Verbindungsschicht 2b weggelassen worden, während in Fig. 3 die innere Schicht bei 2a weggelassen wurde. Es versteht sich, daß auch mit der in Fig. 1 dargestellten Ausführungsform eines Schlauches entsprechende Verbindungsanordnungen, wie in Fig. 2 oder Fig. 3 zu sehen, möglich sind.

Anwendungsgemäß haben die Konnektoren oder Einbauteile 3 wegen z.B. Dichtigkeitsanforderungen bei der Konnektion oder während der Sterilisation kompliziertere Strukturen und Konturen mit hoher Maßhaltigkeit, die sich bei der Sterilisation nicht verändern dürfen und die in den vorliegenden Abbildungen nicht weiter dargestellt sind.

Für die Konnektoren/Einbauteile 3 werden Konstruktionskunststoffe mit vorzugsweise hohem E-Modul und einem Erweichungspunkt deutlich über 121°C eingesetzt. Dabei bestehen die Konnektoren oder Einbauteile 3 vorzugsweise aus Polypropylen-Homopolymerisaten, Polypropylenrandom-Copolymerisaten mit geringem Ethylengehalt, Polycarbonat oder Polymethylpenten. Bei den Teilen selbst handelt es sich hauptsächlich um spritz-oder blasgeformte Teile, wobei im Rahmen der Erfindung im Konnektor 3 ein gewisser Anteil des in der Verbindungsschicht 2a und/oder 2b des Schlauches enthaltenden Materials mit schwindender Formbeständigkeit bei 121°C enthalten sein kann.

Folgende Versuche erläutern die Erfindung eingehender:

Es wurde eine Verbindungstechnik hergestellt aus einem Lippenschlauch und einem Konnektor, wobei die Einzelteile folgende Spezifikationen aufweisen:
1.
   - Lippenschlauch:: - Ø_{A} = 8 mm
   - Ø₁ = 6 mm
   - Aufbau von außen nach innen:
      Siegelschicht aus 50 Gew.-% PP-R und 50 Gew.-% SIS mit einer Dicke von 40 µm;
      Basisschicht aus PP (Shore D ≤32) und einer Dicke von 920 µm; und
      Konnektionsschicht aus 60 Gew.-% SEBS-compound und 40 Gew.-% SEBS/SEB mit einer Dicke von 40 µm
2.
   - Konnektor:: - aus PP-Homopolymer;
   - Schaft konisch, Ø steigt von 6,2 auf 6,8 mm;
   - Schaftoberfläche glatt
3. Konnektor wird in Lippenschlauch gepreßt und bei 121°C, 20 min Haltezeit sterilisiert.

### Versuch 1: Aufblasversuch bei 121°C

Das sterilisierte Lippenschlauch/Konnektor-System wird in einem Wärmeschrank (121 °C) nach 10 min Aufheizzeit mit Innendruck beaufschlagt. Zur Beurteilung der Dichtheit der Verbindung hängt der Konnektor/Schlauchsitz in einem Tauchbad (Glycerin).

| Innendruck | Haltezeit | Beobachtung |
|---|---|---|
| p = 0.1 bar | 15 min | keine Luftblasen sichtbar |
| p = 0.25 bar | 15 min | keine Luftblasen sichtbar |
| p = 0,5 bar | 10 s | nach ca. 1 min Schlauch geplatzt keine Luftblasen sichtbar |

### Versuch 2: Aufblasversuch bei Raumtemperatur

Das sterilisierte Lippenschlauch/Konnektor-System wird bei Raumtemperatur (20°C) mit Innendruck beaufschlagt.
Zur Beurteilung der Dichtheit der Verbindung hängt der Konnektor/Schlauchsitz in einem Tauchbad (H₂O).

| Innendruck | Haltezeit | Beobachtung |
|---|---|---|
| p = 0.2 bar | 15 min | keine Luftblasen sichtbar |
| p = 0.5 bar | 15 min | keine Luftblasen sichtbar |
| p = 0.75 bar | 15 min | keine Luftblasen sichtbar |
| p = 1,0 bar | 15 min | keine Luftblasen sichtbar |
| p = 1,5 bar | 30 min | keine Luftblasen sichtbar |
| p = 2.0 bar | 10 min | Luftblasen sichtbar |

### Versuch 3: Durchführung wie Versuch 1, jedoch ohne vorherige Sterilisation des Lippenschlauch-/Konnektorsystems.

| Innendruck | Haltezeit | Beobachtung |
|---|---|---|
| p = 0.1 bar | 15 min | keine Luftblasen sichtbar |
| p = 0.2 bar | 15 min | keine Luftblasen sichtbar |
| p = 0.5 bar | 1 min | Konnektor rutscht aus Schlauch |

### Versuch 4: Zugversuch bei 70°C

Das sterilisierte Lippenschlauch-/Konnektorsystem wird in einer, mit Heizkammer versehenen Zugprüfmaschine an Schlauch- und Konnektorende gespannt.
Die Heizkammer ist auf 70°C temperiert.
Die Probenvorheizzeit beträgt 10 min.

| Zugversuch: | Abstand Spannbacken | 50 mm |
|---|---|---|
| | Vorspannkraft Fᵥ | 2 N |
| | Prüfgeschwindigkeit: | 200 mm/min |

| Probe | Fₘₐₓ [N] |
|---|---|
| 1 | 63.68 |
| 2 | 56.35 |
| 3 | 63.78 |

Weitere Ausführungsformen und Vorteile der Erfindung ergeben sich aus den nachfolgenden Patentansprüchen.

## Patentansprüche

1. Verbindungsanordnung aus einem Konnektor, Einbauteil oder Anschlußstutzen (erstes Einzelteil) eines medizinischen Systems und einem Non-PVC-Mehrschichtschlauch (zweites Einzelteil) für medizinische Zwecke, bei welcher wenigstens ein Abschnitt einer Oberfläche aus einem ersten Kunststoffmaterial des ersten Einzelteils mit wenigstens einem Abschnitt einer Oberfläche aus einem zweiten Kunststoffmaterial des zweiten Einzelteils in einem Kontaktbereich, der zumindest Teile der sich berührenden Oberflächen des ersten und zweiten Einzelteils umfaßt,
eine feste und dichte Verbindung miteinander bilden, welche durch Inkontaktbringen der miteinander zu verbindenden Oberflächen des ersten und zweiten Einzelteils anschließende Wärmebehandlung der in Kontakt befindlichen Oberflächen der Einzelteile und nachfolgende Abkühlung erhältlich ist,
**dadurch gekennzeichnet, daß**
das erste Kunststoffmaterial wenigstens ein Polymer aufweist, welches bei der Temperatur der Wärmebehandlung formbeständig ist, während das zweite Kunststoffmaterial wenigstens ein Polymer aufweist, welches bei der Temperatur der Wärmebehandlung nicht mehr formbeständig ist und unter Preßkraft zum Fließen neigt, wobei beide Kunststoffmaterialien frei von PVC oder EVA sind und
die Verbindung durch Inkontaktbringen der Oberflächen des ersten und zweiten Einzelteils unter gleichzeitiger Einwirkung einer die Oberflächen gegeneinander pressenden Kraft und Wärmebehandlung der gegeneinander gepreßten Einzelteile erhältlich ist, wobei die Wärmebehandlung ein Hitze-Sterilisationsvorgang ist und die Temperatur der Wärmebehandlung ≥121°C ist und wobei der Non-PVC-Mehrschichtschlauch für medizinische Zwecke wenigstens zwei Schichten aufweist, von denen eine Basisschicht A) aus einem dritten Kunststoffmaterial mit wenigstens einer Konnektionsschicht B) aus dem zweiten Kunststoffmaterial verbunden ist, wobei das dritte Kunststoffmaterial wenigstens ein Polymer aufweist, dessen Formbeständigkeit größer als die Temperatur der Wärmebehandlung ist, während das zweite Kunststoffmaterial wenigstens ein Polymer aufweist, welches unter Konnektionsdruck Fließfähigkeit bei einer Temperatur kleiner oder gleich der Temperatur der Wärmebehandlung aufweist.

2. Verbindungsanordnung nach Anspruch 1, **dadurch gekennzeichnet, daß** die Formbeständigkeit des ersten Kunststoffmaterials bei Temperaturen größer der Temperatur der Wärmebehandlung gegeben und die des zweiten Kunststoffmaterials bei Temperaturen kleiner oder gleich der Temperatur der Wärmebehandlung nicht gegeben ist.

3. Verbindungsanordnung nach einem der vorhergehenden Ansprüche, **dadurch gekennzeichnet, daß** das erste und zweite Einzelteil zu einem medizinischen Beutelsystem für medizinische Lösungen gehören.

4. Verbindungsanordnung nach einem der Ansprüche 1 bis 2 **dadurch gekennzeichnet, daß** das erste und zweite Einzelteil zu einem Schlauchsystem für medizinische Anwendungen gehören.

5. Verbindungsanordnung nach einem der vorhergehenden Ansprüche, **dadurch gekennzeichnet, daß** die verbundenen Einzelteile vollständig aus Kunststoff bestehen.

6. Verbindungsanordnung nach einem der vorhergehenden Ansprüche, **dadurch gekennzeichnet, daß** das erste Einzelteil wenigstens im Kontaktbereich aus Polypropylen (PP), Polycarbonat (PC), einem die Struktureinheiten, die den vorgenannten Homopolymeren zugrundeliegen, aufweisenden Copolymeren und/oder aus Blends, die auf den vorgenannten Polymeren basieren mit bis zu 40 Gew.-% des Kunststoffmaterials, aus dem das zweite Einzelteil im Kontaktbereich besteht, ist.

7. Verbindungsanordnung nach einem der vorhergehenden Ansprüche, **dadurch gekennzeichnet, daß** das erste Einzelteil wenigstens im Kontaktbereich aus einem Blend besteht,
aufweisend
a) 60 bis 100 Gew.-% Polypropylen oder Polycarbonat,
und
b) 40 - 0 Gew.-%, Polyethylen-Copolymer oder SEBS/SEPS mit Diblockanteil oder Polyetherblockamid.

8. Verbindungsanordnung nach einem der vorhergehenden Ansprüche, **dadurch gekennzeichnet, daß** das erste Einzelteil wenigstens im Kontaktbereich aus Polypropylen-Copolymer besteht.

9. Verbindungsanordnung nach einem oder mehreren der Ansprüche 1 bis 2 und 5 bis 8, **dadurch gekennzeichnet, daß** das zweite Einzelteil ein Non-PVC-Schlauch ist, mit wenigstens einer Konnektionsschicht B) aus einem zweiten Kunststoffmaterial, wobei das zweite Kunststoffmaterial wenigstens ein Polymer aufweist, welches unter Konnektionsdruck Fließfähigkeit bei einer Temperatur kleiner oder gleich der Temperatur der Wärmebehandlung aufweist.

10. Verbindungsanordnung nach Anspruch 9, **dadurch gekennzeichnet, daß** die Polymerzusammensetzung der Konnektionsschicht B bei ≤121°C nicht mehr formbeständig ist.

11. Verbindungsanordnung nach Anspruch 9 oder 10, **dadurch gekennzeichnet, daß** die Schichten des Non-PVC-Schlauches ohne zusätzlichen Haftvermittler zusammenhaften und im wesentlichen frei von Weichmachern, Antiblockmitteln, Antistatika sowie anderen Füllstoffen sind.

12. Verbindungsanordnung nach einem der Ansprüche 9 bis 11, **dadurch gekennzeichnet, daß** das zweite Kunststoffmaterial der Konnektionsschicht B) ein Polymeres oder ein Blend ist aus
a) 40% - 100% PE-Copolymer
und
0% - 60% SEPS/SEP,
b) 40% - 100% PE-Copolymer
und
0% - 60% SEBS/SEB,
c) 100% SEPS/SEP,
d) 40% - 100% SEBS/SEB
0 - 60% SEBS
oder
e) 100% Polyetherblockamid.

13. Verfahren zum Verbinden zweier zu einem medizinischen System gehörender Einzelteile ohne zusätzliche Hilfsmittel nur durch Hitzebehandlung, bei welchem unter Ausbildung eines Kontaktbereichs, der durch sich berührende Flächen des ersten und zweiten Einzelteils definiert ist, wenigstens ein Abschnitt einer Oberfläche des ersten Einzelteils aus einem ersten Kunststoffmaterial mit wenigstens einem Abschnitt einer Oberfläche des zweiten Einzeiteils aus einem zweiten Kunststoffmaterial zu einer Verbindungsanordnung zusammengefügt wird, anschließend die zusammengefügte Verbindungsanordnung zur Ausbildung einer festen und dichten Verbindung zwischen erstem und zweitem Einzelteil einer Wärmebehandlung unterzogen und nachfolgend abgekühlt wird, **dadurch gekennzeichnet, daß** man ein erstes Kunststoffmaterial verwendet, das wenigstens ein Polymer aufweist, welches bei der Temperatur der Wärmebehandlung formbeständig ist, während man ein zweites Kunststoffmaterial verwendet, das wenigstens ein Polymer aufweist, welches bei der Temperatur der Wärmebehandlung nicht mehr formbeständig ist und zum Fließen neigt, wobei beide Kunststoffmaterialien frei von PVC und EVA sind und wobei erstes und zweites Einzelteil unter Ausbildung eines Preßsitzes zusammengefügt werden, wobei das zweite Kunststoffmaterial bei der Temperatur der Wärmebehandlung unter Einwirkung einer die Oberflächen im Kontaktbereich gegeneinander pressenden Kraft zum Fließen neigt, wobei man als Wärmebehandlung einen Sterilisationsvorgang vornimmt und die Wärmebehandlung bei einer Temperatur von ≥121°C durchführt und wobei es sich beim zweiten Einzelteil um einen Non-PVC-Mehrschichtschlauch handelt, wobei der Non-PVC-Mehrschichtschlauch für medizinische Zwecke wenigstens zwei Schichten aufweist, von denen eine Basisschicht A) aus einem dritten Kunststoffmaterial mit wenigstens einer Konnektionsschicht B) aus dem zweiten Kunststoffmaterial verbunden ist, wobei das dritte Kunststoffmaterial wenigstens ein Polymer aufweist, dessen Formbeständigkeit größer als die Temperatur der Wärmebehandlung ist, während das zweite Kunststoffmaterial wenigstens ein Polymer aufweist, welches unter Konnektionsdruck Fließfähigkeit bei einer Temperatur kleiner oder gleich der Temperatur der Wärmebehandlung aufweist.

14. Verfahren nach Anspruch 13, **dadurch gekennzeichnet, daß** man ein erstes Einzelteil einsetzt, das wenigstens im Kontaktbereich aus Polypropylen (PP), Polycarbonat (PC), einem die Struktureinbeiten, die den vorgenannten Homopolymeren zugrundeliegen, aufweisenden Copolymeren und/oder aus Blends, die auf den vorgenannten Polymeren basieren, besteht.

15. Verfahren nach einem der Ansprüche 13 oder 14, **dadurch gekennzeichnet, daß** man Einzelteile die zu einem Beutelsystem für medizinische Lösungen gehören, verbindet.

16. Verfahren nach einem der Ansprüche 13 oder 14, **dadurch gekennzeichnet, daß** man als erstes Einzelteil einen Konnektor oder ein Einbauteil aus Kunststoff und als zweites Einzelteil einen coextrudierten Non-PVC-Schlauch miteinander verbindet.

17. Verfahren nach einem der Ansprüche 13 bis 16, **dadurch gekennzeichnet, daß** man einen coextrudierten Non-PVC-Mehrschichtschlauch, dessen Konnektionsschicht aus Polyetherblockamid besteht und einem Konnektor oder ein Einbauteil verwendet, das aus Polycarbonat besteht.

18. Verfahren nach einem der Ansprüche 13 bis 16, **dadurch gekennzeichnet, daß** man einen coextruierten Non-PVC-Mehrschichitschlauch dessen Konnektionsschicht aus Polyethylen-Copolymer, SEPS oder SEBS mit Diblockanteil oder Gemischen daraus besteht und einen Konnektor oder Einbauteile verwendet, der oder die aus Polypropylen besteht oder Polyethylen-Copolymer oder Styrol-Ethylen-Butylen (Propylen)-Kautschuk gemischtes Polyproplen.

## Claims

1. A connection structure comprising a connector, built-in component or connection socket (first component) of a medical system and a non-PVC multi-layer hose (second component) for medical purposes, wherein at least one section of a surface consisting of a first plastic material of the first component forms a permanent, tight connection with at least one section of a surface consisting of a second plastic material of the second component in a contact area, which includes at least parts of the surfaces of the first and second components in contact with one another,
which connection may be obtained by bringing into contact the surfaces of the first and second components to be connected to one another, subsequent heat treatment of the surfaces of the components in contact and subsequent cooling,
**characterised in that**
the first plastic material comprises at least one polymer which is dimensionally stable at the temperature of the heat treatment, whilst the second plastic material comprises at least one polymer which is no longer dimensionally stable at the temperature of the heat treatment and tends to flow under a pressing force, the two plastic materials being free from PVC and EVA,
and
the connection may be obtained by bringing into contact the surfaces of the first and second components with the simultaneous action of a force pressing the surfaces against one another and heat treatment of the components pressed against one another,
the heat treatment being a heat sterilisation process
and
the temperature of the heat treatment being ≥ 121° C
and
wherein the non-PVC multi-layer hose for medical purposes comprises at least two layers, whereof a base layer A) consisting of a third plastic material is connected to at least one connection layer B) consisting of the second plastic material, the third plastic material comprising at least one polymer whose dimensional stability is greater than the temperature of the heat treatment, whilst the second plastic material comprises at least one polymer which, under the connection pressure, exhibits flowability at a temperature less than or equal to the temperature of the heat treatment.

2. The connection structure according to claim 1, **characterised in that** the dimensional stability of the first plastic material is present at temperatures greater than the temperature of the heat treatment and that of the second plastic material is not present at temperatures less than or equal to the temperature of the heat treatment.

3. The connection structure according to any one of the preceding claims, **characterised in that** the first and second component belong to a medical bag system for medical solutions.

4. The connection structure according to any one of claims 1 to 2, **characterised in that** the first and second component belong to a hose system for medical applications.

5. The connection structure according to any one of the preceding claims, **characterised in that** the connected components consist entirely of plastic.

6. The connection structure according to any one of the preceding claims, **characterised in that** the first component consists, at least in the contact area, of polypropylene (PP), polycarbonate (PC), a copolymer comprising the structural units on which the aforementioned homopolymers are based and/or blends which are based on the aforementioned polymers, with up to 40 % by weight of the plastic material of which the second component consists in the contact area.

7. The connection structure according to any one of the preceding claims, **characterised in that** the first component consists, at least in the contact area, of a blend, comprising
a) 60 to 100 % by weight of polypropylene or polycarbonate, and
b) 40 to 0 % by weight of polyethylene copolymer or SEBS/SEPS with a di-block section or polyether block amide.

8. The connection structure according to any one of the preceding claims, **characterised in that** the first component consists, at least in the contact area, of polypropylene copolymer.

9. The connection structure according to one or more of claims 1 to 2 and 5 to 8, **characterised in that** the second component is a non-PVC hose, with at least one connection layer B) consisting of a second plastic material, the second plastic material comprising at least one polymer which, under the connection pressure, exhibits flowability at a temperature less than or equal to the temperature of the heat treatment.

10. The connection structure according to claim 9, **characterised in that** the polymer composition of connection layer B is no longer dimensionally stable at ≤ 121° C.

11. The connection structure according to claim 9 or 10, **characterised in that** the layers of the non-PVC hose adhere to one another without an additional bonding agent and are essentially free from plasticisers, anti-blocking agents, anti-static agents and other fillers.

12. The connection structure according to any one of claims 9 to 11, **characterised in that** the second plastic material of connection layer B) is a polymer or a blend of
a) 40% - 100% PE copolymer
and
0% - 60% SEPS/SEP,
b) 40% - 100% PE copolymer
and
0% - 60% SEBS/SEB,
c) 100% SEPS/SEP
d) 40% - 100% SEBS/SEB
0% - 60% SEBS
or
e) 100% polyether block amide

13. A process for connecting two components belonging to a medical system, without additional processing aids solely by heat treatment, wherein, with the formation of a contact area which is defined by surfaces of the first and second component in contact with one another, at least one section of a surface of the first component consisting of a first plastic material is joined together with at least one section of a surface of the second component consisting of a second plastic material to form a connection structure, the assembled connection structure then undergoes a heat treatment to form a permanent and tight connection between the first and second component and is subsequently cooled, **characterised in that** use is made of a first plastic material which comprises at least one polymer, which is dimensionally stable at the temperature of the heat treatment, whilst a second plastic material is used which comprises at least one polymer, which is no longer dimensionally stable at the temperature of the heat treatment and tends to flow, wherein both plastic materials are free from PVC and EVA and wherein the first and second components are joined together to constitute an interference fit, wherein the second plastic material tends to flow at the temperature of the heat treatment under the action of a force pressing the surfaces against one another in the contact area, wherein a sterilisation process is carried out as a heat treatment and the heat treatment is carried out at a temperature of ≥ 121° C and wherein the second component is a non-PVC multi-layer hose, wherein the non-PVC multi-layer hose for medical purposes comprises at least two layers, whereof a base layer A) consisting of a third plastic material is connected to a least one connection layer B) consisting of the second plastic material, wherein the third plastic material comprises at least one polymer whose dimensional stability is greater than the temperature of the heat treatment, whilst the second plastic material comprises a polymer which, under the connection pressure, exhibits flowability at a temperature less than or equal to the temperature of the heat treatment.

14. The process according to claim 13, **characterised in that** use is made of a first component which consists, at least in the contact area, of polypropylene (PP), polycarbonate (PC), a copolymer comprising structural units forming the basis of the aforementioned homopolymers and/or of blends which are based on the aforementioned polymers.

15. The process according to any one of claims 13 or 14, **characterised in that** components belonging to a bag system for medical solutions are connected.

16. The process according to any one of claims 13 or 14, **characterised in that** a connector or built-in component of plastic, as the first component, and a coextruded non-PVC hose, as the second component, are connected together.

17. The process according to any one of claims 13 to 16, **characterised in that** use is made of a coextruded non-PVC multi-layer hose, whose connection layer consists of polyether block amide, and a connector or built-in component consisting of polycarbonate.

18. The process according to any one of claims 13 to 16, **characterised in that** use is made of a coextruded non-PVC multi-layer hose, whose connection layer consists of polyethylene copolymer, SEPS or SEBS with a di-block section or blends thereof, and a connector or built-in components which consist of polypropylene or polyethylene copolymer or styrene-ethylene-butylene (propylene) rubber blended polypropylene.

## Revendications

1. Agencement de connexion constitué par un connecteur, par une pièce nécessaire pour le montage ou par une tubulure de raccordement (première partie individuelle) d'un système médical et par un tuyau flexible multicouche exempt de PVC (deuxième partie individuelle) pour des objets médicaux, dans lequel au moins une section d'une surface constituée d'une première matière synthétique de la première partie individuelle avec au moins une section d'une surface constituée d'une deuxième matière synthétique de la deuxième partie individuelle dans une zone de mise en contact qui comprend au moins des parties des surfaces qui se touchent des première et deuxième parties individuelles, forment ensemble une liaison résistante et étanche, que l'on obtient par la mise en contact des surfaces à relier des première et deuxième parties individuelles, par traitement thermique ultérieur des surfaces des parties individuelles se trouvant en contact et par refroidissement ultérieur, **caractérisé en ce que** la première matière synthétique présente au moins un polymère qui possède une stabilité dimensionnelle à la température du traitement thermique, tandis que la deuxième matière synthétique présente au moins un polymère qui ne possède plus de stabilité dimensionnelle à la température du traitement thermique et qui manifeste une tendance au fluage sous l'influence d'une force de pression, les deux matières synthétiques étant exemptes de PVC ou de EVA et la liaison par mise en contact des surfaces des première et deuxième parties individuelles étant obtenues sous l'effet simultané d'une force qui presse les surfaces l'une contre l'autre et d'un traitement thermique des parties individuelles pressées l'une contre l'autre, dans lequel le traitement thermique est un procédé de stérilisation à la chaleur et la température du traitement thermique est ≥ 121 °C et dans lequel le tuyau flexible multicouche exempt de PVC pour des objets médicaux présente au moins deux couches, dont une couche de base A) d'une troisième matière synthétique est combinée avec au moins une couche de connexion B) composée de la deuxième matière synthétique, dans lequel la troisième matière synthétique présente au moins un polymère dont la stabilité dimensionnelle est supérieure à la température du traitement thermique, tandis que la deuxième matière synthétique présente au moins un polymère qui, sous la pression du raccordement, présente une aptitude au fluage à un température qui est inférieure ou égale à la température du traitement thermique.

2. Agencement de connexion selon la revendication 1, **caractérisé en ce que** la stabilité dimensionnelle de la première matière synthétique est présente à des températures supérieures à la température du traitement thermique, tandis que celle de la deuxième matière synthétique ne l'est pas à des températures inférieures ou égales à la température du traitement thermique.

3. Agencement de connexion selon l'une quelconque des revendications précédentes, **caractérisé en ce que** les première et deuxième parties individuelles font partie d'un système de sac médical pour des solutions médicales.

4. Agencement de connexion selon l'une quelconque des revendications 1 à 2, **caractérisé ce que** les première et deuxième parties individuelles font partie d'un système de tuyau flexible pour des applications médicales.

5. Agencement de connexion selon l'une quelconque des revendications précédentes, **caractérisé en ce que** les parties individuelles reliées sont constituées complètement de matières synthétiques.

6. Agencement de connexion selon l'une quelconque des revendications précédentes, **caractérisé en ce que** la première partie individuelle, au moins dans la zone de contact, est constituée de polypropylène (PP), de polycarbonate (PC), d'un copolymère présentant des unités de structure qui sont à base des homopolymères précités et/ou de mélanges à base des polymères précités avec la matière synthétique dont est constituée la deuxième partie individuelle dans la zone de contact, jusqu'à concurrence de 40 % en poids.

7. Agencement de connexion selon l'une quelconque des revendications précédentes, **caractérisé en ce que** la première partie individuelle est constituée, au moins dans la zone de contact, d'un mélange présentant
a) du polypropylène ou du polycarbonate à concurrence de 60 à 100 % en poids ; et
b) un copolymère de polyéthylène ou du SEBS/SEPS comprenant une fraction biséquencée ou un polyéther amide séquencé, à concurrence de 40 à 0 % en poids.

8. Agencement de connexion selon l'une quelconque des revendications précédentes, **caractérisé en ce que** la première partie individuelle est constituée, au moins dans la zone de contact, d'un copolymère de polypropylène.

9. Agencement de connexion selon une ou plusieurs des revendications 1 à 2 et 5 à 8, **caractérisé en ce que** la deuxième partie individuelle est un tuyau flexible exempt de PVC, comprenant au moins une couche de connexion B constituée d'une deuxième matière synthétique, la deuxième matière synthétique présentant au moins un polymère qui présente, dans des conditions de pression de connexion, une aptitude au fluage à une température inférieure ou égale à la température du traitement thermique.

10. Agencement de connexion selon la revendication 9, **caractérisé en ce que** la composition polymère de la couche de connexion B ne manifeste plus de stabilité dimensionnelle à une température ≤ 121 °C.

11. Agencement de connexion selon la revendication 9 ou 10, **caractérisé en ce que** les couches du tuyau flexible exempt de PVC adhèrent l'une à l'autre en l'absence d'un agent adhésif supplémentaire et sont essentiellement exemptes de plastifiants, d'agents s'opposant à l'adhérence par contact réciproque, d'agents antistatiques et d'autres matières de charge.

12. Agencement de connexion selon la revendication 9 à 11, **caractérisé en ce que** la deuxième matière synthétique de la couche de connexion B) est un polymère ou un mélange constitué par
a) un copolymère de PE à concurrence de 40 % à 100 % et du SEPS/SEP à concurrence de 0 % à 60 %,
b) un copolymère de PE à concurrence de 40 % à 100 % et du SEBS/SEB à concurrence de 0 % à 60 %,
c) du SEPS/SEP à concurrence de 100 %,
d) du SEBS/SEB à concurrence de 40 % à 100 %, du SEBS à concurrence de 0 à 60 %, ou
e) du polyétheramide séquencé à concurrence de 100 %.

13. Procédé pour la connexion de deux parties individuelles faisant partie d'un système médical, dans lequel, en formant une zone de contact qui est définie par des surfaces qui se touchent des première et deuxième parties individuelles, on joint au moins une section d'une surface de la première partie individuelle constituée d'une première matière synthétique à au moins une section d'une surface de la deuxième partie individuelle constituée d'une deuxième matière synthétique pour obtenir un agencement de connexion, on soumet ensuite à un traitement thermique l'agencement de connexion qui a fait l'objet d'un jointement pour réaliser une connexion résistante et étanche entre les première et deuxième parties individuelles et ensuite on refroidit, **caractérisé en ce qu'**on utilise une première matière synthétique qui présente au moins un polymère qui possède une stabilité dimensionnelle à la température du traitement thermique, tandis que qu'on utilise une deuxième matière synthétique qui présente au moins un polymère qui ne possède plus de stabilité dimensionnelle à la température du traitement thermique et qui manifeste une tendance au fluage sous l'influence d'une force de pression, les deux matières synthétiques étant exemptes de PVC ou de EVA, les première et deuxième parties individuelles étant jointes l'une à l'autre en formant un ajustage serré, la deuxième matière synthétique manifestant une tendance au fluage à la température du traitement thermique sous l'effet d'une force qui presse les surfaces l'une contre l'autre dans la zone de contact, dans lequel on met en oeuvre un procédé de stérilisation à titre de traitement thermique, et on effectue le traitement thermique à une température ≥ 121 °C, et dans lequel, dans le cas de la deuxième partie individuelle, il s'agit d'un tuyau flexible multicouche exempt de PVC dans lequel le tuyau flexible multicouche exempt de PVC pour des objets médicaux présente au moins deux couches, dont une couche de base A) d'une troisième matière synthétique est combinée avec au moins une couche de connexion B) composée de la deuxième matière synthétique, dans lequel la troisième matière synthétique présente au moins un polymère dont la stabilité dimensionnelle est supérieure à la température du traitement thermique, tandis que la deuxième matière synthétique présente au moins un polymère qui, sous la pression du raccordement, présente une aptitude au fluage à un température qui est inférieure ou égale à la température du traitement thermique

14. Procédé selon la revendication 13, **caractérisé en ce qu'**on met en oeuvre une première partie individuelle au moins dans la zone de contact, est constituée de polypropylène (PP), de polycarbonate (PC), d'un copolymère présentant des unités de structure qui sont à base des homopolymères précité et/ou de mélanges à base des polymères précités.

15. Procédé selon l'une quelconque des revendications 13 ou 14, **caractérisé en ce qu'**on procède à la connexion de deux parties individuelles qui font partie d'un système de sac médical pour des solutions médicales.

16. Procédé selon l'une quelconque des revendications 13 ou 14, **caractérisé ce qu'**on procède à la connexion conjointe, à titre de première partie individuelle, d'un connecteur ou d'une pièce en matière synthétique nécessaire à la construction et, à titre de deuxième partie individuelle, d'un tuyau flexible coextrudé exempt de PVC.

17. Procédé selon l'une quelconque des revendications 13 à 16, **caractérisé en ce qu'**on utilise un tuyau flexible multicouche coextrudé exempt de PVC, dont la couche de connexion est constituée d'un polyétheramide séquencé et un connecteur ou une pièce nécessaire à la construction, qui est constitué de polycarbonate.

18. Procédé selon l'une quelconque des revendications 13 à 16, **caractérisé en ce qu'**on utilise un tuyau flexible multicouche coextrudé exempt de PVC dont la couche de connexion est constituée d'un copolymère de polyéthylène, de SEPS ou de SEBS possédant une fraction biséquencée et un connecteur ou des pièces nécessaires à la construction qui est ou qui sont constituées de polypropylène ou d'un copolymère de polyéthylène ou encore d'un polypropylène mixte de styrène-éthylène-caoutchouc butylène (propylène).
